# EUROPEAN PATENT SPECIFICATION

(11) **EP 2 338 519 B1**
(45) Date of publication and mention of the grant of the patent: **17.02.2016**
(21) Application number: 09811296.4
(22) Date of filing: 04.09.2009
(51) Int. Cl.: C12N 15/11, A61K 31/7105, A61K 31/713, A61K 31/07

(54) **AGENT FOR TREATING MYELOFIBROSIS**
MITTEL ZUR BEHANDLUNG VON MYELOFIBROSE
AGENT DE TRAITEMENT D'UNE MYÉLOFIBROSE

(30) Priority: 05.09.2008 JP 2008228338
(43) Date of publication of application: 29.06.2011
(73) Proprietor: Nitto Denko Corporation, Ibaraki-shi Osaka 567-8680 (JP)
(72) Inventor: NIITSU, Yoshiro, Sapporo-shi Hokkaido 064-0823 (JP); MATSUNAGA, Takuya, Sapporo-shi Hokkaido 060-8556 (JP)
(74) Representative: Hoffmann Eitle
(86) International application number: PCT/JP2009/004369
(87) International publication number: WO 2010/026766

(56) References cited:
- EP-A1- 1 842 557
- EP-A1- 2 135 600
- EP-A1- 2 258 395
- WO-A1-2006/068232
- JP-T- 2004 523 236
- KIM D G ET AL: "Retinol-encapsulated low molecular water-soluble chitosan nanoparticles", INTERNATIONAL JOURNAL OF PHARMACEUTICS, ELSEVIER BV, NL, vol. 319, no. 1-2, 17 August 2006 (2006-08-17), pages 130-138, XP027972444, ISSN: 0378-5173 [retrieved on 2006-08-17]
- YOUNG-IL JEONG ET AL: "Polyion complex micelles composed of all-trans retinoic acid and poly (ethylene glycol)-grafted-chitosan", JOURNAL OF PHARMACEUTICAL SCIENCES, vol. 95, no. 11, 1 November 2006 (2006-11-01), pages 2348-2360, XP55036964, ISSN: 0022-3549, DOI: 10.1002/jps.20586
- WATANABE, J. ET AL.: 'Treatment of idiopathic myelofibrosis employing siRNA for heat shock protein 47 (siRNA/HSP47) encapsulated in liposomes' BLOOD vol. 110, no. 11, 2007, pages 235B - ABS.#464, XP008136547
- MARCUCCI FABRIZIO ET AL: "Active targeting with particulate drug carriers in tumor therapy: fundamentals and recent progress", DRUG DISCOVERY TODAY, ELSEVIER, RAHWAY, NJ, US, vol. 9, no. 5, 1 March 2004 (2004-03-01), pages 219-228, XP002510380, ISSN: 1359-6446, DOI: 10.1016/S1359-6446(03)02988-X
- TORCHILIN ET AL: "Drug Targeting", EUROPEAN JOURNAL OF PHARMACEUTICAL SCIENCES, ELSEVIER, AMSTERDAM, NL, vol. 11, no. supp2, 1 January 2000 (2000-01-01), pages s81-s91, XP002202027, ISSN: 0928-0987, DOI: 10.1016/S0928-0987(00)00166-4
- SCHUBERT S ET AL: "Ribozyme- and deoxyribozyme-strategies for medical applications", CURRENT DRUG TARGETS, BENTHAM SCIENCE PUBLISHER, US, vol. 5, no. 8, 1 November 2004 (2004-11-01), pages 667-681, XP009180178, ISSN: 1389-4501
- ACHENBACH TATJANA V ET AL: "Oligonucleotide-based knockdown technologies: antisense versus RNA interference", CHEMBIOCHEM - A EUROPEAN JOURNAL OF CHEMICAL BIOLOGY, WILEY VCH, WEINHEIM, DE, vol. 4, no. 10, 6 October 2003 (2003-10-06), pages 928-935, XP002324625, ISSN: 1439-4227, DOI: 10.1002/CBIC.200300708

### SEQUENCE LISTING

<110> NITTO DENKO CORPORATION
<120> Agent for treating myelofibrosis
<130> PCT2380ND
<150> JP 2008-228338
   <151> 2008-09-05
<160> 13
<170> PatentIn version 3.5
<210> 1
   <211> 27
   <212> RNA
   <213> Artificial sequence
<220>
   <223> siRNA-A sense strand
<400> 1
   uggaugggaa agaugcagaa gaaggag 27
<210> 2
   <211> 27
   <212> RNA
   <213> Artificial sequence
<220>
   <223> siRNA-A antisense strand
<400> 2
   ccuucuucug caucuuuccc auccaau 27
<210> 3
   <211> 27
   <212> RNA
   <213> Artificial sequence
<220>
   <223> siRNA-B sense strand
<400> 3
   ugucugagug gguauuuuua gacagag 27
<210> 4
   <211> 27
   <212> RNA
   <213> Artificial sequence
<220>
   <223> siRNA-B antisense strand
<400> 4
   cugucuaaaa auacccacuc agacaau 27
<210> 5
   <211> 27
   <212> RNA
   <213> Artificial sequence
<220>
   <223> siRNA-C sense strand
<400> 5
   gaugcgagau gaguuguaga guccaag 27
<210> 6
   <211> 27
   <212> RNA
   <213> Artificial sequence
<220>
   <223> siRNA-C antisense strand
<400> 6
   uggacucuac aacucaucuc gcaucau 27
<210> 7
   <211> 27
   <212> RNA
   <213> Artificial sequence
<220>
   <223> siRNA-D sense strand
<400> 7
   cagaacugcc cauccuuaaa augauag 27
<210> 8
   <211> 27
   <212> RNA
   <213> Artificial sequence
<220>
   <223> siRNA-D antisense strand
<400> 8
   aucauuuuaa ggaugggcag uucugau 27
<210> 9
   <211> 27
   <212> RNA
   <213> Artificial sequence
<220>
   <223> siRNA-E sense strand
<400> 9
   gagacaagau gcgagaugag uuguaag 27
<210> 10
   <211> 27
   <212> RNA
   <213> Artificial sequence
<220>
   <223> siRNA-E antisense strand
<400> 10
   uacaacucau cucgcaucuu gucucau 27
<210> 11
   <211> 27
   <212> RNA
   <213> Artificial sequence
<220>
   <223> Scramble siRNA sense strand
<400> 11
   cgauucgcua gaccggcuuc auugcag 27
<210> 12
   <211> 27
   <212> RNA
   <213> Artificial sequence
<220>
   <223> Scramble siRNA antisense strand
<400> 12
   gcaaugaagc cggucuagcg aaucgau 27
<210> 13
   <211> 2273
   <212> DNA
   <213> Mus musculus
<400> 13
   gagcgattgc cctgtcggtc ccggagcagc ccagcccggc ccaggtgagg ggcgggacgg 60
   ccgggcgatt tggggttgcg cattggccaa gggggaacgg atcgtccaaa gcttggggtc 120
   ctgacacagg tgggcagtga aagatcggac ttggaactgt cacttgcctg ggttccctgg 180
   agctgcggtc tagtacgcgg actgcctggt aaaccctcac aggtcctctg tggtgcacac 240
   agcccacctc ccagccatgc gctctctcct tctgggcacc ttatgcctct tggccgtggc 300
   cctggcagcc gaggtgaaga aacccctaga ggcggcagcc cctggtactg cggagaagct 360
   aagttccaag gcgaccacac tggcagagcg cagcacaggc ctggccttca gcctatatca 420
   ggcgatggcc aaagaccagg cggtggagaa catcctcctg tcacccttgg tggtggcctc 480
   atccctgggt cttgtgtcac tgggtggtaa agccaccaca gcgtcgcagg cgaaggcagt 540
   gctgagcgct gagaagctgc gcgatgagga ggtgcacacg gggctgggtg agctgctccg 600
   ctccctcagc aactccactg cgcgcaacgt gacctggaaa ctgggcagcc gcctgtacgg 660
   gcccagctcc gtgagcttcg ccgatgactt cgtgcgcagc agcaagcaac actacaactg 720
   cgaacactcc aagatcaact tccgagacaa gcgcagcgcc ctgcagtcca tcaacgagtg     780
   ggcctcgcag accacggacg gcaagctgcc tgaggtcacc aaggatgtgg agcgcacgga     840
   tggggcactg cttgtgaacg ccatgttctt taagccacac tgggatgaga ggtttcacca     900
   caggatggtg gacaaccgtg gcttcatggt gacccgctcc tatactgtgg gtgttacgat     960
   gatgcaccgg acaggcctgt acaactacta tgacgacgag aaggagaagc tgcagatggt    1020
   ggagatgccc ctggctcaca agctctccag cctcatcatc ctcatgcccc accatgtgga    1080
   gccgctcgag cgcttggaga agctgctgac caaggagcag ctgaaggcct ggatgggaaa    1140
   gatgcagaag aaggctgtcg ccatctccct gcccaagggc gtggtggagg tgacccatga    1200
   cctgcagaaa catctggcag gactgggcct gaccgaagcc atcgacaaga acaaggcaga    1260
   cctatcgcgc atgtctggca agaaggacct gtacctggcc agtgtgttcc acgccactgc    1320
   cttcgagtgg gacaccgagg gcaacccctt tgaccaagac atctacgggc gcgaggagct    1380
   gcgcagcccc aagctgttct atgccgacca ccccttcatc ttcctggtgc gagataatca    1440
   gagcggctcc ttgctcttca ttggccgcct ggtccggccc aagggagaca agatgcgaga    1500
   tgagttgtag agtccaagag tgggcgtggc acggcaggaa gtagccaaag gttcctgaga    1560
   cacatgggtg ctattgtggg gggaggggga ggcaccaacc ctggatattc cacgggtggg    1620
   ggggggttgg cagtgcaaac cggaattccc atatgtctga gtgggtattt ttagacagaa    1680
   tccactccta agttaggaca tggagcccag atactatgat accaaattca ggggtgtcac    1740
   agccattttg ttctgccctg caagttttag atccaatctg cctcaacagt caatcagtgt    1800
   tcatatttat ggccaggcat tttatctgtt agacaatcga gttgggggta gggcagccta    1860
   gctctttttg tcaccatgcc ccgagccctc ctcagtcttc ctgttcaccc ctcccccagc    1920
   tccctttaac cgcaaaacta ggtgctgtgg ccccagaact gcccatcctt aaaatgatcc    1980
   ttgcccagcg gtgggagctg gagacagcgc atggaggggg ctccctggca tacctgccct    2040
   agaactgtta tgttgggcat cacagtcatg aacttttgtt tttctccctt ttttagtttt    2100
   ttcaaagatg gggggagggg aaatatgagc ctttgttgct ttcaaaacga gaacagtttg    2160
   tacaagtttt tttttgaata aaacttttcc aatgaaatgt tacaggtgtg tgaaagcaga    2220
   ttaaagaaag aggttcccat atcattcttt ctaaaaaaaa aaaaaaaaaa aaa            2273

## Description

### [Technical Field]

The present invention relates to a composition for use in the treatment of myelofibrosis, comprising a drug that controls the activity or growth of an extracellular matrix-producing cell in bone marrow and a substance delivery carrier targeted to extracellular matrix-producing cells in bone marrow, as well as to a kit for preparing said composition.

### [Background Arts]

Myelofibrosis is a general term referring to diseases which causes an extensive diffuse fibrosis in bone marrow, and includes primary myelofibrosis whose etiology is unknown and secondary myelofibrosis with an underlying disease.
Primary myelofibrosis belongs to a chronic myeloproliferative disorder, being characterized by the involvement of a fibrosis in bone marrow throughout the body and extramedullary hematopoiesis in liver and spleen, as well as the manifestation of leukoerythroblastosis in which immature granulocytes and erythroblasts appear in peripheral blood. The essential of the primary myelofibrosis is considered to be a monoclonal proliferation of hematopoietic cells due to genetic abnormality including Jak2 gene mutation caused at the level of hematopoietic stem cells. Various cytokines produced by the proliferated hematopoietic cells (mainly megakaryocyte) act on bone marrow stromal cells to cause a proliferation of reactive polyclonal bone marrow stromal cells, which leads the fibrosis of bone marrow, osteosclerosis and angiogenesis. This results in characteristic clinical symptoms such as an ineffective hematopoiesis, an appearance of dacryocytes in peripheral blood, leukoerythroblastosis, and an extramedullary hematopoiesis causing a splenomegaly. Approximately 40 % of the primary myelofibrosis have gene mutation in Jak2, a tyrosine kinase essential for signal transduction of cytokines, resulting in a constitutive activation of Jak2 even in the absence of a cytokine stimulation. Apart from Jak2, there are a few cases with genetic mutation in c-mp1 (a thrombopoietin receptor). It is currently considered to be difficult to cure primary myelofibrosis by drug therapy, and an allogeneic transplantation of hematopoietic stem cells is the sole curative therapy. However, the mortality rate associated with transplant is as high as 25 to 48 %, limiting the total survival rate to around 50 %. Recently, the utility of nondisruptive transplant of bone marrow stem cells (mini-transplant) with less treatment-associated toxicity has been highlighted, yet only a limited number of cases has been studied and their long-term prognoses are yet to be known.

As drug therapy, although being palliative, the effectiveness of anabolic hormones such as danazol and Primobolan, angiogenesis inhibitor such as thalidomide and lenalidomide, anti-tumor drug such as hydroxycarbamide, anagrelide, imatinib, 2-chlorodeoxyadenosine, melphalan, busulfan and etoposide, and other drugs such as erythropoietin, for anemia, thrombocytopenia and splenomegaly has been shown (see Non-Patent Literatures 1 and 2).

On the other hand, secondary myelofibrosis is those which occur secondary to a disease such as acute myeloid leukemia, acute lymphocytic leukemia, chronic myeloid leukemia, polycythemia vera, primary thrombocythemia, myelodysplastic syndrome, multiple myeloma, malignant lymphoma, carcinoma, systemic lupus erythematosus and progressive systemic sclerosis, or radiation, and shows a similar bone marrow image to primary myelofibrosis. Treatment for the secondary myelofibrosis is focused on improving the underlying disease. However, many of these underlying diseases are difficult to be radically cured. Thus, there is a strong need for alleviating the adverse effect due to myelofibrosis itself.

In those circumstances, a great deal of investigation has been made for the development of myelofibrosis therapeutics. As a result, there have been reports that successes to a certain extent were provided in animal models of myelofibrosis or in clinical trials by, for example, inhibitors of a tyrosine kinase JAK2V617F, TGF-β inhibitors such as soluble TGF-β receptor, NFκB inhibitors such as bortezomib, DNA methyltransferase inhibitors such as decitabine, histone deacetylase inhibitors such as trichostatin A, VEGF inhibitors such as PTK787/ZK222584 and bevacizumab (see Non-Patent Literature 1) and certain types of anti-human lymphocyte antibody (see Patent Literature 1). Non-Patent Literature 3 is a paper on the treatment of idiopathic myelofibrosis employing siRNA for heat shock protein 47 (siRNA/HSP47) encapsulated in liposomes. However, none of these drugs are satisfactory, and development of further agent for treating myelofibrosis has been longed.

[Patent Literature 1] JP A No. 8-002799
[Patent Literature 2] WO 2006/068232
[Non-Patent Literature 1] Hematology Am Soc Hematol Educ Program. 2007;2007:346-54
[Non-Patent Literature 2] The Journal of the Japanese Society of Internal Medicine, Vol. 96, No. 7, July 10, 2007, pp.1398-1404
[Non-Patent Literature 3] WATANABE, J., et al. in ASH Annual Meeting Abstracts. 2007, vol. 110, no. 11, p. 4646.

### [Disclosure of Invention]

### [Problems to be Solved by the Invention]

The present invention is aimed to provide a novel agent for treating myelofibrosis.

### [Means for Solving the Problems]

The inventors have discovered, through the exploration for a novel therapeutic agent for myelofibrosis, that myelofibrosis could effectively be treated by administering a composition in which an extracellular matrix production inhibitor is carried by a carrier comprising a retinoid, thereby completed the invention.
While it has been known that a carrier comprising vitamin A can deliver a drug to stellate cells that store vitamin A (see Patent Literature 2), its relation to myelofibrosis has been completely unknown to date. Nor has there been any report that myelofibrosis could be treated by a composition comprising as an active ingredient an extracellular matrix production inhibitor. Therefore, the current findings are quite surprising.

Namely, the present invention relates to the following:
1. A composition for use in the treatment of myelofibrosis, comprising a drug that controls the activity or growth of an extracellular matrix-producing cell in bone marrow, and a carrier comprising a retinoid as a targeting agent, wherein the drug that controls the activity or growth of the extracellular matrix-producing cell in bone marrow is selected from the group consisting of a drug that suppresses the expression of the extracellular matrix component and is selected from an siRNA, a ribozyme and antisense nucleic acid or a vector expressing these or an siRNA, a ribozyme, an antisense nucleic acid which target molecules involved in the production or secretion of said extracellular matrix component, and a vector that expresses said siRNA, ribozyme, and antisense nucleic acid.
2. The composition for use according to item 1, wherein the molecule involved in the production or secretion of the extracellular matrix component is HSP47.
3. The composition for use according to item 1 or 2, wherein the retinoid comprises retinol.
4. The composition for use according to any one of items 1 to 3, wherein the retinoid content is 0.2 - 20 wt % of the entire carrier.
5. The composition for use according to any one of items 1 to 4, wherein the carrier has a form, which is selected from the group consisting of a macromolecular micelle, a liposome, an emulsion, microspheres and nanospheres.
6. The composition for use according to any one of items 1 to 5, wherein the carrier has a form of a liposome, and the molar ratio of the retinoid to the lipid contained in the liposome is 8:1 - 1:4.
7. The composition for use according to any one of items 1 to 6, which is provided in a form that can be prepared immediately before use.
8. A kit for preparing a composition for use according to any one of items 1 to 7, comprising one or more containers that comprises either singly or in combination the drug that controls the activity or growth of an extracellular matrix-producing cell in bone marrow, the retinoid, and if necessary, a carrier-constituent substance other than the retinoid,
further comprising instructions for administrating the composition as specified in item 1,
wherein the drug that controls the activity or growth of the extracellular matrix-producing cell in bone marrow is selected from the group consisting of a drug that suppresses the expression of the extracellular matrix component and is selected from an siRNA, a ribozyme and antisense nucleic acid or a vector expressing these or an siRNA, a ribozyme, an antisense nucleic acid which target molecules involved in the production or secretion of said extracellular matrix component, and a vector that expresses said siRNA, ribozyme, and antisense nucleic acid.

### [The effects of the Invention]

While the exact mechanism of action of the composition for treating myelofibrosis of the present invention has not yet been completely clarified, the mechanism is considered as follows: with the composition, retinoid functions as a targeting agent to extracellular matrix-producing cells in bone marrow such as bone marrow fibroblasts, and delivers active ingredients such as pharmaceutical agents that control activity or growth of extracellular matrix-producing cells in bone marrow to such cells, thereby exhibiting the effect against myelofibrosis.
Since active ingredients can be efficiently delivered to a site of action and further to a target cell, by using the carrier of the present invention, the treatment, suppression of progression, and prevention of onset of myelofibrosis, in particular primary myelofibrosis, the treatment of which has been difficult to date, are enabled; thus, the present carrier significantly contributes to the human medicine and veterinary medicine.
Furthermore, since the composition of the present invention comprises as an active agent a drug that controls the activity or growth of an extracellular matrix-producing cell as specified in appended claim 1, whose effectiveness to myelofibrosis has not been known, it can treat myelofibrosis by a different mechanism from those currently known. Therefore, it is expected to ameliorate pathologic conditions which could not be treated by drugs of conventional mechanism, and to increase the therapeutic effect of the combined use with those conventional drugs.
Moreover, the carrier to be used in the present invention can be combined with any pharmaceutical drugs as specified in appended claim 1 (for example, existing therapeutic agents for myelofibrosis) to increase their action efficiency; therefore, it is also advantageous for its broad range of application in terms of formulation, facilitating the production of effective therapeutic agents.

### [Brief Description of the Drawings]

[Fig. 1]

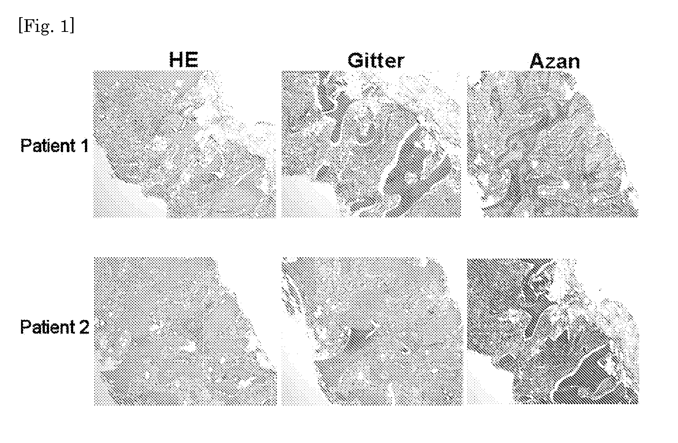

   Fig. 1 shows photographs showing bone marrow images of ideopathic myelofibrosis patients. Both Patient 1 and Patient 2 showed trabecular thickening by HE staining (left column), reticular fiber hyperplasia by Gitter staining (central column) and collagen deposition by Azan staining (right column).
[Fig. 2]

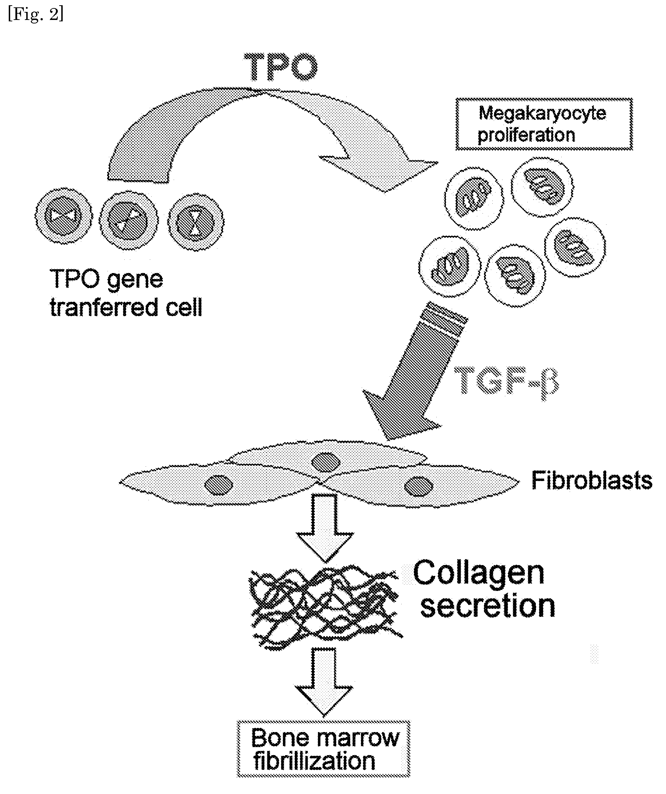

   Fig. 2 is a diagram showing the pathogenesis of a myelofibrosis model mouse.
[Fig. 3]

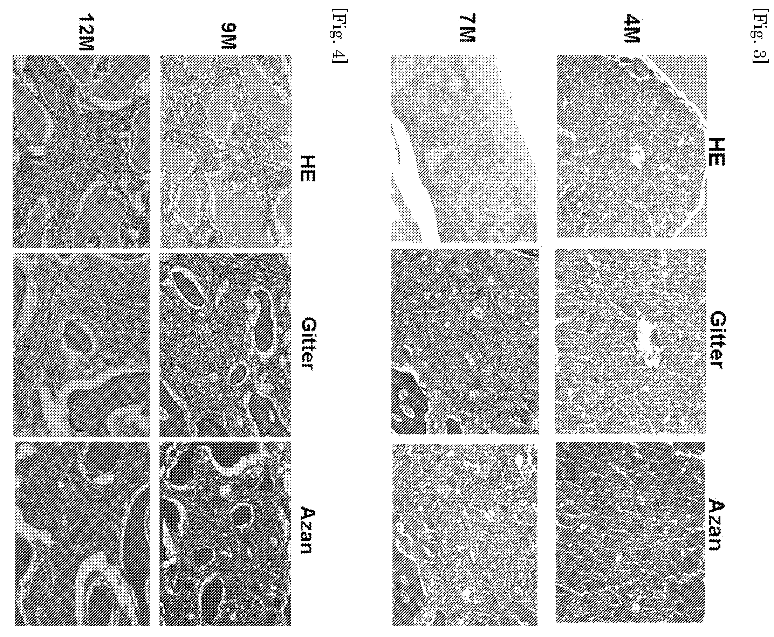

   Fig. 3 shows photographs showing bone marrow images of TPO transgenic mice of 4-and 7-months old. The left column shows HE staining images, the central column shows Gitter staining images and the right column shows Azan staining images.
[Fig. 4]
   Fig. 4 shows photographs showing bone marrow images of TPO transgenic mice of 9-and 12-months old. The left column shows HE staining images, the central column shows Gitter staining images and the right column shows Azan staining images.
[Fig. 5]

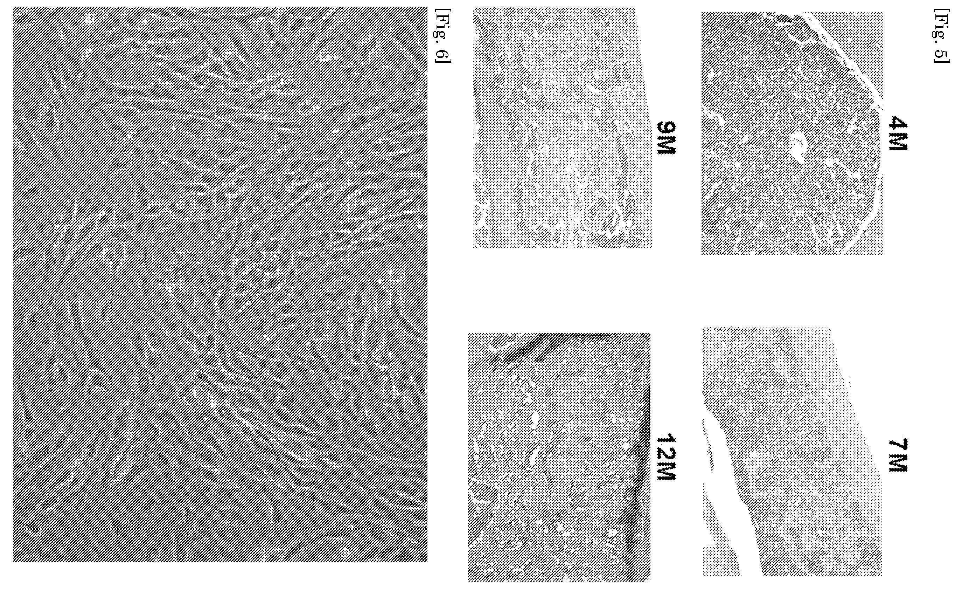

   Fig. 5 shows photographs showing the transition in trabecular thickening in TPO transgenic mice. The top left panel is a HE staining image of bone marrow at 4 months old (4M), the top right panel is at 7 months old (7M), bottom left paten is at 9 months old (9M), and the bottom right panel is at 12 months old (12M).
[Fig. 6]
   Fig. 6 is a photograph showing cell morphology of TPO mouse-derived primary-cultured bone marrow fibroblasts observed by an inverted microscope (magnification x400).
[Fig. 7]

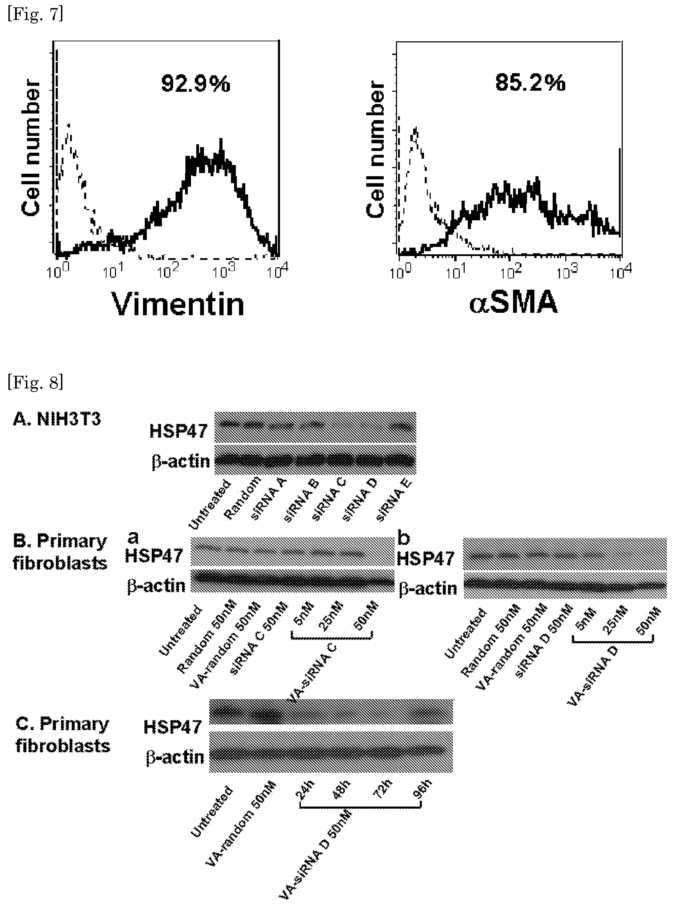

   Fig. 7 shows diagrams showing the results of flow cytometric analyses of Vimentin and α-SMA expressions in TPO mouse-derived primary-cultured bone marrow fibroblasts. The vertical axes indicate cell number.
[Fig. 8]
   Fig. 8 shows western blot images showing the effect of various siRNA HSP47 on HSP47 expression in NIH3T3 (A) and primary cultures of TPO mouse-derived bone marrow fibroblasts (Primary fibroblasts; B and C).
[Fig. 9]

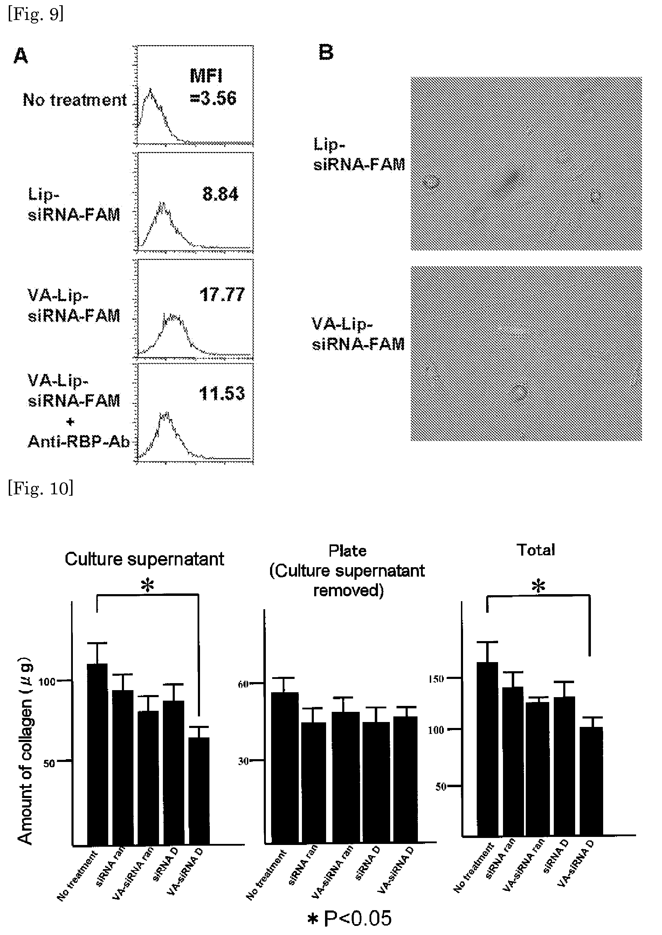

   Fig. 9 shows diagrams showing the effect of vitamin A (VA) on the introduction of a liposome-embedded HSP47 siRNA (Lip-siRNA) into TPO mouse-derived primary-cultured bone marrow fibroblasts. (A) and (B) show results of flow cytometric analyses and fluorescence microscopic images, respectively.
[Fig. 10]
   Fig. 10 shows diagrams showing the effect of siRNA HSP47 on collagen secretion by TPO mouse-derived primary-cultured bone marrow fibroblasts.
[Fig. 11]

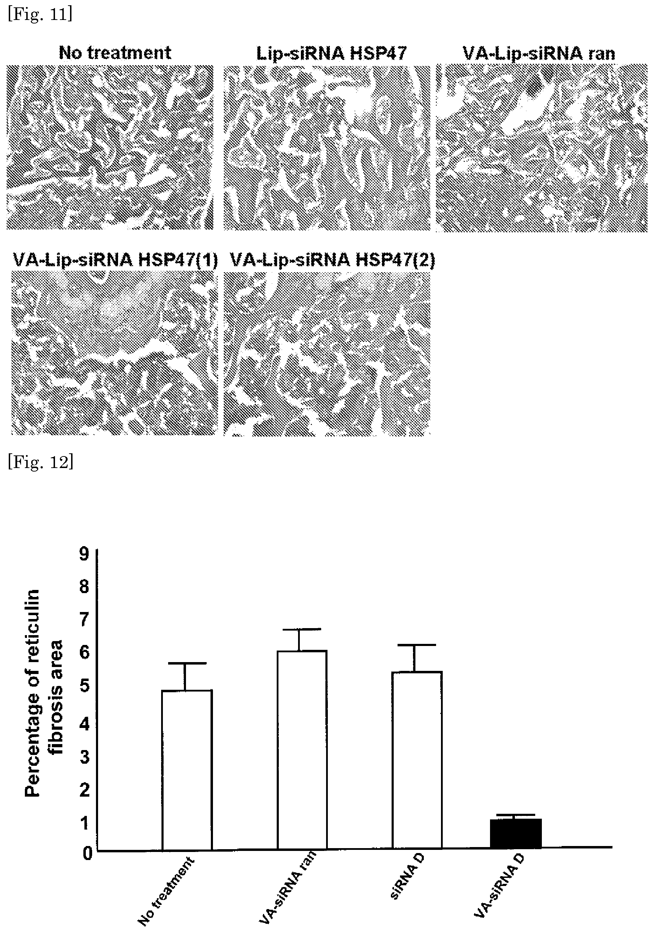

   Fig. 11 shows microscopic images of Gitter staining samples showing an in vivo effect of siRNA HSP47 on bone marrow fibrillization in TPO mice.
[Fig. 12]
   Fig. 12 is a graph showing the quantification of the level of improvement in reticular fiber hyperplasia in TPO mice by siRNA HSP47 by image analyses. The vertical axis indicates the ratio of the dots for reticular fiber against entire dots in each field.
[Fig. 13]

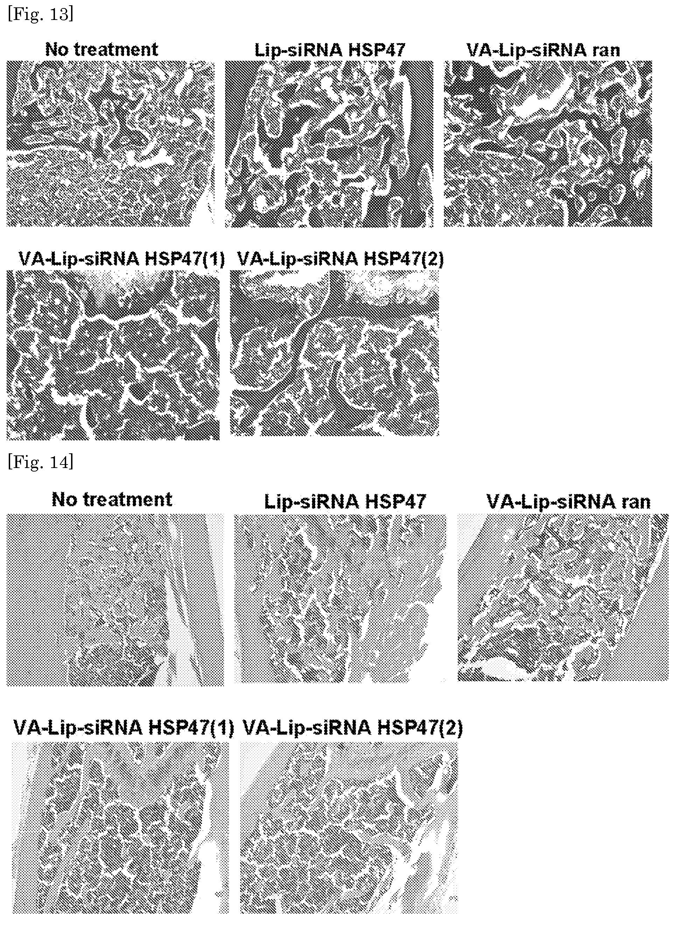

   Fig. 13 shows microscopic images of Azan staining samples showing an in vivo effect of siRNA HSP47 on bone marrow fibrillization in TPO mice.
[Fig. 14]
   Fig. 14 shows microscopic imaging of HE staining samples showing an in vivo effect of siRNA HSP47 on bone marrow fibrillization in TPO mice.

### [Description of Embodiments]

The present invention relates to a substance delivery carrier for the delivery of the substance to an extracellular matrix-producing cell in bone marrow, comprising a retinoid as a targeting agent.
In the present invention, the extracellular matrix-producing cell in bone marrow is not particularly limited as long as it is a cell being present in bone marrow and having a capability of producing extracellular matrix, and it typically includes a bone marrow fibroblast. A bone marrow fibroblast is characterized by the expression of α-SMA (alpha-smooth muscle actin). The bone marrow fibroblast in the present invention is one of those identified, e.g., by immunostaining using detectably-labeled anti-α-SMA antibodies.

The retinoid to be used in the present invention is not particularly limited as long as it promotes delivery of a substance to an extracellular matrix-producing cell in bone marrow, and examples thereof include retinoid derivatives such as retinol (vitamin A), etretinate, tretinoin, isotretinoin, adapalene, acitretine, tazarotene, and retinol palmitate, as well as vitamin A analogues such as fenretinide (4-HPR, 4-hydroxyphenylretinamide) and bexarotene.
The retinoid to be used in the present invention is one of whose which promote a specific delivery of a substance to an extracellular matrix-producing cell in bone marrow. The mechanism of the promotion of substance delivery by retinoid has not yet been completely clarified; however, for example, it is considered that a retinoid which has specifically bound to a retinol-binding protein (RBP) is taken into an extracellular matrix-producing cell in bone marrow through a certain receptor present on the surface of this cell.
A retinoid is a member of a class of compounds having a skeleton in which four isoprenoid units are bonded in a head-to-tail manner (see G. P. Moss, "Biochemical Nomenclature and Related Documents," 2nd Ed. Portland Press, pp. 247-251 (1992)). Vitamin A is a generic descriptor for a retinoid that qualitatively shows the biological activity of retinol. Retinoid that can be used in the present invention is not particularly limited, and examples thereof include retinoid derivatives such as retinol, retinal, retinoic acid, an ester of retinol and a fatty acid, an ester of an aliphatic alcohol and retinoic acid, etretinate, tretinoin, isotretinoin, adapalene, acitretine, tazarotene and retinol palmitate, and vitamin A analogues such as fenretinide (4-HPR) and bexarotene.
Of these, retinol, retinal, retinoic acid, an ester of retinol and a fatty acid (such as retinyl acetate, retinyl palmitate, retinyl stearate and retinyl laurate) and an ester of an aliphatic alcohol and retinoic acid (such as ethyl retinoate) are preferable from the viewpoint of efficiency of specific delivery of a substance to extracellular matrix-producing cells in bone marrow.
All retinoid isomers including cis-trans isomers are included in the scope of the present invention. The retinoid may be substituted with one or more substituents. The retinoid in the present invention includes a retinoid in an isolated form as well as in a form of a solution or mixture with a medium that can dissolve or retain the retinoid.

The carrier to be used in the present invention may be constituted from the retinoid on its own or may be constituted by binding the retinoid to a carrier-constituent component other than the retinoid, or by enclosing the retinoid in a carrier-constituent component other than the retinoid. Therefore, the carrier to be used in the present invention may comprise a carrier-constituent component other than the retinoid. Such a component is not particularly limited, and any component known in the medicinal and pharmaceutical fields may be used, but those that can enclose the retinoid or can bind to the retinoid are preferable.
Examples of such a component include a lipid, for example, a phospholipid such as glycerophospholipid, a sphingolipid such as sphingomyelin, a sterol such as cholesterol, a vegetable oil such as soybean oil or poppy seed oil, a mineral oil, and a lecithin such as egg-yolk lecithin, but the examples are not limited thereto. Among them, those that can form a liposome are preferable, for example, a natural phospholipid such as lecithin, a semisynthetic phospholipid such as dimyristoylphosphatidylcholine (DMPC), dipalmitoylphosphatidylcholine (DPPC), or distearoylphosphatidylcholine (DSPC), and dioleylphosphatidylethanolamine (DOPE), dilauroylphosphatidylcholine (DLPC), and cholesterol.

A particularly preferred component is a component that can avoid capture by the reticuloendothelial system, and examples thereof include cationic lipids such as N-(α-trimethylammonioacetyl)-didodecyl-D-glutamate chloride (TMAG), N,N',N",N"'-tetramethyl-N,N',N",N"'-tetrapalmitylspermine (TMTPS), 2,3-dioleyloxy-N-[2(sperminecarboxamido)ethyl]-N,N-dimethyl-1-propanaminium trifluoroacetate (DOSPA), N-[1-(2,3-dioleyloxy)propyl]-N,N,N-trimethylammonium chloride (DOTMA), dioctadecyldimethylammonium chloride (DODAC), didodecylammonium bromide (DDAB), 1,2-dioleyloxy-3-trimethylammoniopropane (DOTAP), 3β-[N-(N',N'-dimethylaminoethane)carbamoyl] cholesterol (DC-Chol), 1,2-dimyristoyloxypropyl-3-dimethylhydroxyethylammonium (DMRIE), and O,O'-ditetradecanoyl-N-(α-trimethylammonioacetyl)diethanolamine chloride (DC-6-14).

The binding of the retinoid to the carrier for use in the present invention or the enclosing of it therein is also made possible by binding the retinoid to or enclosing it in a carrier constituent other than the retinoid by a chemical and/or physical method. Alternatively, the retinoid can be bound to or enclosed in the carrier for use in the present invention by mixing the retinoid and the carrier constituents other than the retinoid during the preparation of the carrier. The amount of the retinoid bound to or enclosed in the carrier for use in the present invention may be, as a weight ratio in the carrier-constituent components, 0.01% to 100%, preferably 0.2% to 20%, and more preferably 1% to 5%. The retinoid may be bound to or enclosed in the carrier before loading a drug to the carrier; or the carrier, retinoid and drug may simultaneously be mixed; or the retinoid may be admixed with the carrier already carrying the drug, etc. Therefore, the present invention also relates to a process for producing a formulation specific to an extracellular matrix-producing cell in bone marrow, the process comprising a step of binding a retinoid to any existing drug-binding carrier or drug-encapsulating carrier, for example, a liposomal formulation such as DaunoXome®, Doxil, Caelyx®, or Myocet®.

The carrier for use in the present invention may be in any form as long as a desired substance or object can be transported to a target extracellular matrix-producing cell in bone marrow, and although not limited thereto, examples thereof include a macromolecular micelle, a liposome, an emulsion, microspheres, and nanospheres. In the present invention, a liposomal form is preferable among these from the viewpoint of a high delivery efficiency, a wide selection of substances to be delivered, and an ease of formulation, etc., and a cationic liposome including a cationic lipid is particularly preferable. In the case where the carrier is in a form of a liposome, the molar ratio of the retinoid to other constituents of the liposome is preferably 8:1 to 1:4, more preferably 4:1 to 1:2, yet more preferably 3:1 to 1:1, and particularly preferably 2:1, considering the efficiency in retinoid's binding to or enclosure in the carrier.

The carrier for use in the present invention may contain a substance to be transported within its interior, may be attached to the exterior of a substance to be transported, or may be mixed with a substance to be transported, wherein said substance to be transported is the drug specified in appended claim 1, as long as it comprises retinoid in a form such that the retinoid is able to function as a targeting agent. "Function as a targeting agent" herein means that the carrier comprising a retinoid reaches and/or is taken up by the target cell, i.e., an extracellular matrix-producing cell in bone marrow, more rapidly and/or in a larger quantity than with a carrier not comprising the retinoid, and this may easily be confirmed by, for example, adding a labeled carrier or label-containing carrier to a culture of target cells and analyzing the distribution of the label after a predetermined period of time. Structurally, this requirement can be satisfied, for example, if retinoid is at least partially exposed to the exterior of the formulation containing the carrier at the latest by the time it reaches the target cell. Whether or not the retinoid is exposed at the exterior of a formulation can be evaluated by contacting the formulation to a substance that specifically binds to retinoid, such as a retinol-binding protein (RBP), and evaluating its binding to the formulation.

The substance or object that is delivered by the present carrier is the drug specified in appended claim 1, and it preferably has a size such that it can physically move within the body of an organism from the site of administration to the site of lesion where the target cell is present. Therefore, the carrier for use in the present invention can transport the drug specified in appended claim 1. The drug has the property of controlling (e.g. increasing or suppressing) the activity or growth of the target cell.

Therefore, in the present invention, what is delivered by the carrier is "a drug that controls the activity or growth of an extracellular matrix-producing cell in bone marrow" as specified in appended claim 1. The activity of the extracellular matrix-producing cell in bone marrow herein refers to various activities such as secretion, uptake or migration exhibited by an extracellular matrix-producing cell in bone marrow, and in the present invention, in particular, among these, it typically means an activity involved in the onset, progression, and/or recurrence of myelofibrosis. Examples of such activities include, but are not limited to, the production/secretion of a bioactive substance such as gelatinase A and gelatinase B (MMP2 and MMP9, respectively) and angiotensinogen, and of an extracellular matrix component such as collagen, proteoglycan, tenascin, fibronectin, thrombospondin, osteopontin, osteonectin and elastin.

Therefore, the drug that controls the activity or growth of an extracellular matrix-producing cell in bone marrow may be any drug that directly or indirectly suppresses the physical, chemical and/or physiological actions of said cell related to the onset, progression and/or recurrence of myelofibrosis, provided it is a drug that suppresses the expression of the extracellular matrix component and is selected from an siRNA, a ribozyme and antisense nucleic acid (including RNA, DNA, PNA (peptide nucleic acid), or a composite thereof), or a vector expressing these or an siRNA, a ribozyme, an antisense nucleic acid which target molecules involved in the production or secretion of said extracellular matrix component, and a vector that expresses said siRNA, ribozyme, and antisense nucleic acid and particularly preferably, inhibitors against Heat Shock Protein 47 (HSP47), inter alia, siRNA against HSP47.

The substance or object delivered by the carrier for use in the present invention may or may not be labeled. Labeling enables monitoring of the success or failure of transport, or increases and decreases in target cells, etc., and is particularly useful at the testing/research level. A label may be selected from any label known to a person skilled in the art such as, for example, any radioisotope, magnetic material, substance that binds to a labeled substance (e.g. an antibody), fluorescent substance, fluorophore, chemiluminescent substance, and enzyme, etc.
In the present invention, "to an extracellular matrix-producing cell in bone marrow" or "for the delivery to an extracellular matrix-producing cell in bone marrow" means that it is suitable to use to the extracellular matrix-producing cells as a target cell, and this includes, for example, that it is possible to deliver a substance to this cell, more rapidly, efficiently, and/or in a larger quantity than to other cells, for example, normal cells. For example, the carrier for use in the present invention can deliver a substance to an extracellular matrix-producing cell in bone marrow at a rate and/or efficiency of 1.1 times or more, 1.2 times or more, 1.3 times or more, 1.5 times or more, 2 times or more, or even 3 times or more compared with other cells.

The present invention thus relates to a composition for treating myelofibrosis, the composition comprising the drug that controls the activity or growth of the extracellular matrix-producing cell in bone marrow as specified in appended claim 1, and the present invention also relates to a use of the drug that controls the activity or growth of the extracellular matrix-producing cell in bone marrow as specified in appended claim 1 in the production of said compositions. The drug may be contained in the composition alone or together with a pharmaceutically acceptable carrier. The composition of the present invention may be targeted to an extracellular matrix-producing cell in bone marrow, which will be the target, for efficient delivery to said cell. The way of targeting is not particularly limited as long as it includes the addition of a retinoid. Accordingly, the present invention includes a retinoid as a targeting agent, and more preferably, includes a carrier comprising above-mentioned retinoid as a targeting agent.

In the present invention, myelofibrosis includes primary myelofibrosis as well as secondary myelofibrosis. Secondary myelofibrosis includes, without limitation, those which occur secondary to a disease such as acute myeloid leukemia, acute lymphoid leukemia, chronic myeloid leukemia, polycythemia vera, primary thrombocythemia, myelodysplastic syndrome, multiple myeloma, malignant lymphoma, carcinoma, systemic lupus erythematosus and progressive systemic sclerosis, or to radiation. Myelofibrosis in the present invention can be diagnosed by any methods known in the art. The most characteristic pathology of myelofibrosis is a fibrillization of bone marrow, and this can be determined to some extent by a failure in collecting of bone marrow aspirate by bone marrow aspiration ("dry tap"). A definitive diagnosis is made by confirming the fibrillization of bone marrow and/or an increase in trabecula by bone marrow biopsy (see Fig. 1). Primary myelofibrosis may further manifest anemia, hepatosplenomegaly, appearances of leukoerythroblastosis, poikilocytes such as dacryocytes, blast cells, macrothrombocytes and megakaryocytes in peripheral blood, an increase in serum LDH, an increase in hepatosplenic uptake by bone marrow scintigraphy, occasional bleeding tendency, abdominal bloating, fever, general malaise, loss of body weight, etc. In secondary myelofibrosis, the symptoms of the underlying disease often come to the fore. Specific symptoms of an underlying disease are well known by those skilled in the art.

In the composition of the present invention, as long as the retinoid contained in the carrier is present in a mode such that it functions as a targeting agent, the carrier may contain a substance to be delivered within its interior, may be attached to the exterior of a substance to be delivered, or may be mixed with a substance to be delivered, wherein the substance is the drug as specified in appended claim 1. Therefore, depending on the administration route and the manner in which the drug is released, etc., the composition may be covered with an appropriate material such as, for example, an enteric coating or a timed-disintegrating material, or may be incorporated into an appropriate drug release system.
The composition of the present invention may be administered via various routes including both oral and parenteral routes, and examples thereof include, but are not limited to, oral, intravenous, intramuscular, subcutaneous, local, intrapulmonary, intra-airway, intratracheal, intrabronchial, nasal, rectal, intraarterial, intraportal, intraventricular, intramedullar, intra-lymph-node, intralymphatic, intrabrain, intrathecal, intracerebroventricular, transmucosal, percutaneous, intranasal, intraperitoneal, and intrauterine routes, and it may be formulated into a dosage form suitable for each administration route. Such a dosage form and formulation method may be selected as appropriate from any known dosage forms and methods (see e.g. Hyojun Yakuzaigaku (Standard Pharmaceutics), Ed. by Yoshiteru Watanabe et al., Nankodo, 2003).
Examples of dosage forms suitable for oral administration include, but are not limited to, powder, granule, tablet, capsule, liquid, suspension, emulsion, gel, and syrup, and examples of the dosage form suitable for parenteral administration include injections such as an injectable solution, an injectable suspension, an injectable emulsion, and an injection to be prepared immediately before use. Formulations for parenteral administration may be in a form such as an aqueous or nonaqueous isotonic sterile solution or suspension.

The composition of the present invention may further comprise one or more of any other drugs that may cure myelofibrosis or alleviate the onset, progress and/or recurrence and/or symptoms thereof. The examples of such drugs include, without limitation, for example, anabolic hormones such as danazol and Primobolan, angiogenesis inhibitor such as thalidomide and lenalidomide, anti-tumor drug such as hydroxycarbamide, anagrelide, imatinib, 2-chlorodeoxyadenosine, melphalan, busulfan and etoposide, erythropoietin, inhibitors of JAK2V617F tyrosine kinase, TGF-β inhibitors such as soluble TGF-β receptor, NFκB inhibitors such as bortezomib, DNA methyltransferase inhibitors such as decitabine, histone deacetylase inhibitors such as trichostatin A, VEGF inhibitors such as PTK787/ZK222584 and bevacizumab, and anti-human lymphocyte antibody described in Patent literature 1 above. When used in combination, the composition of the present invention may be administered simultaneously with, before or after the other drug. The administration route can be the same or different. For example, the composition of the present invention may be administered parenterally, whereas the other drug may be administered orally, etc.

The carrier or the composition of the present invention may be provided in any form, but from the viewpoint of storage stability, it is preferably provided in a form that can be prepared immediately before use, for example in a form such that it can be prepared at a place of medical treatment or in the vicinity thereof by a doctor and/or pharmacist, nurse or other paramedic. In this case, the carrier or the composition of the present invention is provided as one or more containers containing at least one constituent essential for it, and it is prepared prior to use, for example, within 24 hours prior to use, preferably within 3 hours prior to use, and more preferably, immediately prior to use. When preparing, a reagent, a solvent, preparation equipment, etc. that are normally available in the place of preparation may be used as appropriate.

Accordingly, the present invention also relates to a kit for preparing a composition of the present invention, the kit comprising one or more containers that contain singly or in combination a retinoid, and a substance to be delivered, and/or a carrier-constituent substance other than the retinoid, as well as to a constituent that is necessary for the carrier or composition, provided in a form of such kit, wherein the substance to be delivered is the drug as specified in appended claim 1. The kit of the present invention may comprise, in addition to the above, instructions, an electronic recording medium such as a CD or DVD regarding methods for preparing or administrating the carrier and composition of the present invention, etc. Furthermore, the kit of the present invention may comprise all of the constituents for completing the carrier or the composition of the present invention, but need not necessarily to comprise all of the constituents. Accordingly, the kit of the present invention need not comprise a reagent or solvent that is normally available at a place of medical treatment or experimental facility, etc., such as, for example, sterile water, physiological saline or glucose solution.

The composition of the present invention can be used in a method for controlling the activity or growth of an extracellular matrix-producing cell in bone marrow, or a method for treating myelofibrosis, the method comprising administering an effective amount of foregoing composition to a subject in need thereof. The effective amount herein, in a method for treating myelofibrosis, for example, is an amount that suppresses the onset or recurrence of myelofibrosis, alleviates its symptoms, or delays or brings to a halt its progression, and is preferably an amount that prevents the onset or recurrence of myelofibrosis or cures it. It is also preferably an amount that does not cause an adverse effect that exceeds the benefit from administration. Such an amount may appropriately be determined by an in vitro test using cultured cells or by a test in a model animal such as a mouse, rat, dog or pig, and such test methods are well known to a person skilled in the art. Moreover, the dose of the retinoid contained in the carrier and the dose of the drug used in the present invention are known to a person skilled in the art, or may appropriately be determined by the above-mentioned test, etc.

The specific dose of the composition of the present invention may be determined in view of various conditions with respect to the subject in need of the treatment, such as the severity of symptoms, general health condition of the subject, age, body weight, gender of the subject, diet, the timing and frequency of administration, a concurrent medicament, responsiveness to the treatment, and the compliance with the treatment, etc.
The route of administration includes various routes including both oral and parenteral routes, for example, such as oral, intravenous, intramuscular, subcutaneous, local, intrapulmonary, intra-airway, intratracheal, intrabronchial, nasal, rectal, intraarterial, intraportal, intraventricular, intramedullar, intra-lymph-node, intralymphatic, intrabrain, intrathecal, intracerebroventricular, transmucosal, percutaneous, intranasal, intraperitoneal and intrauterine routes.
The frequency of administration varies depending on the properties of the composition to be used and the aforementioned conditions of the subject, and may be, for example, a plurality of times per day (i.e., 2, 3, 4, 5, or more times per day), once a day, every few days (i.e., every 2, 3, 4, 5, 6, or 7 days, etc.), a few times per week (e.g. 2, 3, 4 times, etc. per week), once a week, or every few weeks (i.e., every 2, 3, 4 weeks, etc.).

In the method of the present invention, the term "subject" means any living individual, preferably an animal, more preferably a mammal, and yet more preferably a human individual. In the present invention, the subject may be healthy or affected by myelofibrosis, typically a subject affected by myelofibrosis or at a risk of being affected by myelofibrosis. When prevention of primary myelofibrosis is intended, for example, typical examples include, without limitation, a subject having a gene mutation in Jak2 and/or c-mpl. When prevention of secondary myelofibrosis is intended, typical examples include, without limitation, a subject being affected by a disease such as acute myeloid leukemia, acute lymphoid leukemia, chronic myeloid leukemia, polycythemia vera, primary thrombocythemia, myelodysplastic syndrome, multiple myeloma, malignant lymphoma, carcinoma, systemic lupus erythematosus or progressive systemic sclerosis, or a subject who has undergone irradiation. Furthermore, the term "treatment" includes all types of medically acceptable prophylactic and/or therapeutic intervention for the purpose of the cure, temporary remission or prevention of a disorder. For example, the term "treatment" includes medically acceptable intervention of various purposes, including delaying or halting the progression of myelofibrosis, regression or disappearance of a lesion, prevention of onset and prevention of recurrence of myelofibrosis.

The composition of the present invention can also be used in a method for delivering the drug specified in appended claim 1 to an extracellular matrix-producing cell in bone marrow. This method includes, but is not limited to, for example, a step of loading said drug to be delivered onto the carrier, and a step of administering or adding the carrier carrying the substance to be delivered to an organism or a medium, for example a culture medium, which contains an extracellular matrix-producing cell in bone marrow. These steps may appropriately be achieved according to any known method or such as a method described in the present specification. The delivery method may be combined with another delivery method, for example, another delivery method for targeting bone marrow. Moreover, the method includes an embodiment performed in vitro and an embodiment in which an extracellular matrix-producing cell in bone marrow inside the body is targeted.

The present application also discloses a novel siRNA against mouse HSP47, preferably those targeted to the part selected from position 1130 to position 1145, position 1485 to position 1500, position 1501 to position 1516, position 1654 to position 1678 and position 1951 to position 1978 of SEQ. ID NO: 13. Although methods for designing and producing an siRNA against specific region of a gene for suppressing the expression of said gene are known in the art, it is generally impossible to predict the part of the gene which should be targeted, and this can only be ascertained via experiment. In the present invention, for their strong suppressive effect on HSP47 expression, an siRNA that targets to position 1501 to position 1516, and an siRNA that targets to position s 1951 to position 1978 of SEQ ID NO: 13 are preferred. Some preferred examples of the novel siRNAs for use in the present invention consist of following combinations of sense strand and antisense strand.

Sequences A: a combination of:
5'-UGGAUGGGAAAGAUGCAGAAGAAGGAG-3' (sense, SEQ ID NO:1) and
3'-UAACCUACCCUUUCUACGUCUUCUUCC-5' (antisense, SEQ ID NO:2).

Sequences B: a combination of:
5'-UGUCUGAGUGGGUAUUUUUAGACAGAG-3' (sense, SEQ ID NO:3) and
3'-UAACAGACUCACCCAUAAAAAUCUGUC-5' (antisense, SEQ ID NO:4).

Sequences C: a combination of:
5'-GAUGCGAGAUGAGUUGUAGAGUCCAAG-3' (sense, SEQ ID NO:5) and
3'-UACUACGCUCUACUCAACAUCUCAGGU-5' (antisense, SEQ ID NO:6).

Sequences D: a combination of:
5'-CAGAACUGCCCAUCCUUAAAAUGAUAG-3' (sense, SEQ ID NO:7) and
3'-UAGUCUUGACGGGUAGGAAUUUUACUA-5' (antisense, SEQ ID NO:8).

Sequences E: a combination of:
5'-GAGACAAGAUGCGAGAUGAGUUGUAAG-3'(sense, SEQ ID NO:9) and
3'-UACUCUGUUCUACGCUCUACUCAACAU-5'(antisense, SEQ ID NO:10).

Among these, Sequences C and D are particularly preferred for their strong suppressive effect on HSP47 expression.

The siRNA for use in the present invention may have a naturally occurring RNA structure, or may also have various modifications aimed to improve in vivo stability or binding affinity to the target sequence. Such modification includes, without limitation, a modification by a terminal amino group, thiol group, cholesterol, long-chain alkyl, sugar chain or peptide, etc., a formation of abasic site, an introduction of modified nucleic acid such as a locked nucleic acid (LNA), a peptide nucleic acid (PNA), a nucleotide modified at 2' position of the sugar, for example, 2'-O-alkyl-, 2'-O-alkyl-O-alkyl- or 2'-fluoro-modified nucleotide.
The siRNA for use in the present invention is extremely useful for suppressing HSP47 expression in a mouse and for suppressing collagen production associated with HSP47 expression, and can particularly be suitable for the use in researches, experiments and tests using a mouse.

### [Examples]

### Preparatory Example 1: Confirmation of pathology in myelofibrosis model mice.

A thrombopoietine (TPO) transgenic mouse developed by Dr. Kazuya Shimoda and Dr. Mine Harada (hereinbelow may also referred to as TPO mouse; see Leukemia Research 29: 761-769, 2005) was used as a myelofibrosis model mouse. In this mouse, TPO is excessively produced from TPO gene-transferred cell, leading to the expansion of megakaryocyte in bone marrow. The expanded megakaryocytes excessively produce transforming growth factor-beta (TGF-β), which stimulates bone marrow fibroblasts, promoting the secretion of collagen from the fibroblasts and resulting in bone marrow fibrillization (see Fig. 2).
TPO mice (provided from Kyushu University Animal Center) were bred in normal breeding condition, then euthanized at 4, 7, 9 or 12 months after birth, their bone marrow were collected to make tissue samples, which were stained with hematoxylin-eosin (HE) staining, Gitter staining or Azan staining, respectively, and bone marrow images were observed with an optical microscope. The results are shown in Figs. 3 to 5.

No fibrillization was observed at 4 months old (4M). However, at 7 months old (7M), although trabecular thickening was not apparent (HE), reticular fiber hyperplasia (Gitter) and collagen deposition (Azan) were observed (see Fig. 3).
Both at 9 months old (9M) and 12 months old (12M), trabecular thickening was prominent (HE), reticular fiber hyperplasia (Gitter) and collagen deposition (Azan) were also observed. Moreover, bone marrow fibrillization and trabecular thickening had progressed (exacerbated) at 12M compared with at 9M (see Fig. 4).
In HE sample of TPO mouse bone marrow, trabecular thickening began at 9 months old (9M), which had further exacerbated at 12 months old (see Fig. 5).
Accordingly, the development of the symptoms of myelofibrosis has been confirmed in TPO mice.

### Preparatory Example 2: Production of siRNAs.

siRNAs targeted to mouse HSP47 (HSP47 siRNA) were generated as drugs that suppress the activity of extracellular matrix-producing cells in bone marrow. Specifically, five HSP47 siRNAs (HSP47 siRNA-A to E) having the sequences below and a Random siRNA were generated, which were used in the experiments hereinafter. The HSP47 siRNAs were purchased from iGENE Therapeutics, Inc. (Tokyo), and the target sequences of the HSP47 siRNAs were designed from the database Refseq (GenBank Accession No. NM_009825) registered in November 2006. Random siRNA was also purchased from iGENE Therapeutics, Inc. (product name: dsRNA scramble).

HSP47 siRNA-A:
5'-UGGAUGGGAAAGAUGCAGAAGAAGGAG-3' (sense, SEQ ID NO:1)
3'-UAACCUACCCUUUCUACGUCUUCUUCC-5' (antisense, SEQ ID NO:2)

HSP47 siRNA-B:
5'-UGUCUGAGUGGGUAUUUUUAGACAGAG-3' (sense, SEQ ID NO:3)
3'-UAACAGACUCACCCAUAAAAAUCUGUC-5' (antisense, SEQ ID NO:4)

HSP47 siRNA-C:
5'-GAUGCGAGAUGAGUUGUAGAGUCCAAG-3' (sense, SEQ ID NO:5)
3'-UACUACGCUCUACUCAACAUCUCAGGU-5' (antisense, SEQ ID NO:6)

HSP47 siRNA-D:
5'-CAGAACUGCCCAUCCUUAAAAUGAUAG-3' (sense, SEQ ID NO:7)
3'-UAGUCUUGACGGGUAGGAAUUUUACUA-5' (antisense, SEQ ID NO:8)

HSP47 siRNA-E:
5'-GAGACAAGAUGCGAGAUGAGUUGUAAG-3' (sense, SEQ ID NO:9)
3'-UACUCUGUUCUACGCUCUACUCAACAU-5' (antisense, SEQ ID NO:10)

Random siRNA:
5'-CGAUUCGCUAGACCGGCUUCAUUGCAG-3' (sense, SEQ ID NO:11)
3'-UAGCUAAGCGAUCUGGCCGAAGUAACG-5' (antisense, SEQ ID NO:12)
siRNAs which have been labeled with a fluorescent dye 6'-carboxyfluorescein (6-FAM) at 5' end were also produced.

### Reference Example 3: Properties of primary cultured TPO mouse bone marrow fibroblast.

A primary culture of bone marrow fibroblasts was obtained by culturing the bone marrow cells from TPO mice of 4 to 6 weeks old in MEM (Minimum Essential Medium Eagle, Sigma) supplemented with 15% fetal calf serum (FCS) for 4 weeks.
Fig. 6 shows the cell morphology observed by an inverted microscope. The cells had a spindle shape which is typical for a fibroblast. Fig. 7 shows the results of flow cytometric analyses using respective antibodies to mesenchymal cell markers Vimentin and α-SMA (anti-Vimentin antibody (Santa Cruz Biotechnology) and anti-α-SMA antibody (Santa Cruz Biotechnology)). The expression of both markers was observed, indicating that the cells obtained from the culture were typical bone marrow fibroblasts. The flow cytometer used in the analyses was FACS calibur (Becton Dickinson), and measured data was analyzed using CellQuest software (Becton Dickinson).

### Reference Example 4: Effect of HSP47 siRNA on NIH3T3 cell (mouse fibroblast cell line).

1 x 10⁵ NIH3T3 cells were suspended in Dulbecco's modified Eagle's medium (DMEM, Life Technologies) supplemented with 10% Calf serum (CS), and plated onto 6-well culture plates. After 24 hours, NIH3T3 cells at 50-60% confluency were transfected with HSP47 siRNA, using Lipotrust (Hokkaido System Science Co., Ltd.). Specifically, 20nM Lipotrust and 50nM Random siRNA or HSP47 siRNA were mixed by a vortex and used for transfection. The transfected NIH3T3 cells were cultured for 4 hours in serum-free OPTI-MEM (GIBCO). The NIH3T3 cells were then washed with DMEM, and further cultured for 24 hours in DMEM supplemented with 10% CS, and protein was extracted. The HSP47 expression was then analyzed by Western blotting. Namely, the protein extracted from the NIH3T3 cells was fractioned using 4/20 SDS-polyacrylamide gel electrophoresis (SDS-PAGE) and then transferred to a nitrocellulose membrane. It is probed firstly either with a primary antibody against HSP47 (Stressgen) or a primary antibody against β-actin (Cell Signaling Technology), then further probed with a peroxidase-conjugated secondary antibody (Oncogene Research Product), and finally developed using ECL (Amersham Life Science). The result showed that, at 24 hours after the transfer of HSP47 siRNA into NIH3T3 cells, HSP47 siRNA-C and -D had a stronger effect compared to A, B and E (see Fig. 8A). Accordingly, we used HSP47 siRNA-C and -D as HSP47 siRNA in the experiments thereafter.

### Example 5: Effect of HSP47 siRNA on TPO mouse-derived primary-cultured bone marrow fibroblast.

5 x 10⁵ of TPO mouse-derived primary-cultured bone marrow fibroblasts were suspended in MEM supplemented with 15% FCS, and plated onto 6-well culture plates. After 24 hours, bone marrow fibroblasts at 50-60% confluency were transfected with HSP47 siRNA, using Lipotrust. Namely, 20nM Lipotrust and 50nM Random siRNA or 5-50nM HSP47 siRNA-C or -D were mixed by a vortex and used for transfection, or these were further mixed with 40nM vitamin A (retinol, Sigma) by a vortex, after 5 minutes, and used for transfection. Bone marrow fibroblasts transfected either with vitamin A-conjugated or vitamin A-unconjugated liposome HSP47 siRNA were cultured for 4 hours in serum-free OPTI-MEM. Then these bone marrow fibroblasts were washed with MEM, further cultured for 48 hours in MEM supplemented with 15% FCS, and protein was extracted. In a different experiment, bone marrow fibroblasts transfected with 50nM vitamin A-conjugated liposome HSP47 siRNA-D (VA-Lip-HSP47 siRNA-D; hereinafter "vitamin A-conjugated" and "liposome" may be abbreviated as "VA" and "Lip", respectively) was cultured for 4 hours in serum-free OPTI-MEM and washed with MEM, then further cultured for 24 to 96 hours in MEM supplemented with 15% FCS before extracting protein. Then extracted protein was analyzed for HSP47 expression by Western blotting. Namely, the protein extracted from the bone marrow fibroblasts was fractioned using 4/20 SDS-polyacrylamide gel electrophoresis (SDS-PAGE) and then transferred onto a nitrocellulose membrane. Then, similar to Example 4, it is probed with a primary antibody against HSP47 or β-actin, then further probed with a peroxidase-conjugated secondary antibody, and finally visualized using ECL. The results are shown in Figs. 8B and 8C.

When primary cultured bone marrow fibroblasts were used, neither HSP47 siRNA-C (Fig. 8, a) nor HSP47 siRNA-D (Fig. 8, b) alone showed any effect. However, when conjugated with vitamin A (VA), HSP47 siRNA-C (VA-Lip-HSP47 siRNA-C) was confirmed to express an effect in a concentration of at or above 50nM, while VA-Lip-HSP47 siRNA-D did so at or above than 25nM. Accordingly, it became clear that it is necessary to use VA-Lip-HSP47 siRNA for an efficient transfer of HSP47 siRNA into a primary cultured bone marrow fibroblast, and that VA-Lip-HSP47 siRNA-D had more potent suppressive effect on HSP47 compared with VA-Lip-HSP47 siRNA-C. Thus, we decided to use VA-Lip-HSP47 siRNA-D in the experiments thereafter. Furthermore, it became clear that the effect of VA-Lip-HSP47 siRNA-D (50nM) sustained for 72 hours (see Fig. 8C).

### Example 6: Effect of vitamin A (VA) on the introduction of liposome-embedded HSP47 siRNA (Lip-HSP47 siRNA) in TPO mouse-derived primary-cultured bone marrow fibroblasts.

Primary-cultured bone marrow fibroblasts derived from TPO mouse were transfected with Lip-HSP47 siRNA or VA-Lip-HSP47 siRNA conjugated with 50nM carboxyfluorescein (FAM) (Lip-HSP47 siRNA-FAM or VA-Lip-HSP47 siRNA-FAM) in OPTI-MEM culture medium supplemented with 10% CS, in the presence or absence of 10mg/ml anti- retinol binding protein antibody (anti-RBP-Ab, BD Pharmingen), and after 30 minutes analyzed by flow cytometry. The mean fluorescence intensity (MFI) of transfected cell was measured by FACS calibur and analyzed using CellQuest software (Becton Dickinson). 5 x 10⁵ TPO mouse-derived primary-cultured bone marrow fibroblasts were plated onto a 6-well culture plate. After 24 hours, 50nM VA-Lip-HSP47 siRNA-FAM or Lip-HSP47 siRNA-FAM was added. These cells were cultured for 30 minutes in OPTI-MEM supplemented with 10% FCS, and washed with PBS and fixed with 4% paraformaldehyde thereafter (25 degree C, 15 minutes). Subsequent to fixation, these cells were nuclear-stained with DAPI for 1 minute. The intracellular localization of FAM was evaluated by fluorescence microscope. A typical flow cytometric pattern and a typical result of intracellular localization of FAM-labeled siRNA are shown in Fig. 9A and 9B, respectively. MFI is as indicated in Fig. 9A. It became clear that, compared with Lip-HSP47 siRNA, VA-Lip-HSP47 siRNA exhibited a higher transfection efficiency into a TPO mouse-derived primary-cultured bone marrow fibroblast (A and B). Furthermore, since the introduction of VA-Lip-HSP47 siRNA was partly suppressed by anti-RBP-Ab (A), it was suggested that a part of VA-Lip-HSP47 siRNA uptake could have been made via RBP (Retinol Binding Protein) receptor of the bone marrow fibroblast.

### Example 7: Effect of siRNA HSP47 on collagen secretion from TPO mouse-derived primary-cultured bone marrow fibroblasts.

5 x 10⁵ of primary-cultured bone marrow fibroblasts were suspended in MEM supplemented with 15% FCS, and plated onto 6-well culture plates. After 24 hours, 50nM of either Lip-siRNA Random (siRNA ran), Lip-HSP47 siRNA-D (siRNA D), VA-Lip-siRNA Random (VA-siRNA ran) or VA-Lip-HSP47 siRNA-D (VA-siRNA D) was introduced. After 4 hours, culture medium was replaced by MEM supplemented with 15% FCS, prior to culturing for another 48 hours. The culture medium was then removed and replaced by serum-free OPTI-MEM, before culturing for another 4 hours. After that, firstly the collagen content in the culture supernatant was measured using Sircol™ Collagen Assay kit (Biocolor). Namely, the culture supernatant was mixed with dye solution for 30 minutes, then the solution was centrifuged at 10,000 x g for 10 minutes. After removing unbound dye solution, 1ml of alkaline reagent was added to the bound dye and vortexed for 10 minutes, and the quantification was made based on the absorbance measured by the absorption spectrometer (540nm). Secondly, the amount of collagen deposited on the fibroblasts that are attached to the culture plate was quantified by adding Sirius red dye and measuring the absorbance by absorption spectrometer (540nm).

The result is shown in Fig. 10. Note that "Culture Supernatant" in the figure indicates the collagen content in the culture supernatant of the fibroblasts, whereas "Plate (culture supernatant removed)" indicates the amount of collagen deposited on the fibroblasts after removing the culture supernatant, and "Total" indicates the sum of "Culture Supernatant" and "Plate (culture supernatant removed)", respectively. Data was expressed as a mean of 3 cultures ± SD (*P<0.05).
As a result, it became clear that the amount of collagen secreted into the culture supernatant from the primary-cultured fibroblasts transfected with VA-Lip-HSP47 siRNA-D was significantly smaller compared to the cases of other cells; that in the cells transfected with VA-Lip-HSP47 siRNA-D, the amount of collagen deposition on the culturing plate was smaller but not significant compared to the cases of other cells; and that, in the fibroblasts transfected with VA-Lip-HSP47 siRNA-D, the sum of the amount of collagen secreted into the culture supernatant from the fibroblasts and the amount of collagen deposition on the fibroblasts after removing the culture supernatant was significantly smaller compared to the cases of other cells.

### Example 8: in vivo effect of siRNA HSP47 on bone marrow fibrillization of TPO mice.

We investigated the effect of VA-Lip-HSP47 siRNA-D in vivo to improve bone marrow fibrillization using 7-month-old TPO mice. 12.5mg of HSP47 siRNA-D per mouse was injected intravenously from retro-orbital plexus using tuberculin syringe, every other day to make 4 doses of injection in total. Namely, 12.5mg/mouse of siRNA (8µL) and 12.5nM of Lipotrust (12.5µL), either with or without 25nM of vitamin A (2.5µL), was further topped up with RNAase free PBS to make total 100µL, which is administered to a mouse as one dose. The mice were euthanized 8 days after the start of HSP47 siRNA-D administration, and the bone marrow was collected for preparing tissue samples, each of which were stained with hematoxylin-eosin (HE) staining, Gitter staining or Azan staining, and subjected to bone marrow image observation by an optical microscope. The results are shown in Figs. 11-14.

From the Gitter staining sample after treatment (Fig. 11), it became clear that the reticular fiber hyperplasia in bone marrow was significantly improved in the two mice which had been administered VA-Lip-siRNA HSP47 (VA-Lip-siRNA HSP47 (1) and (2)), compared to the untreated mice (No treatment), Lip-siRNA HSP47-administered mice (Lip-siRNA HSP47) and VA-Lip-siRNA Random-administered mice (VA-Lip-siRNA ran). Moreover, the level of this improvement in reticular fiber hyperplasia by siRNA HSP47 was measured and quantified by KS-400 software (Carl Zeiss). Namely, 10 optical fields were selected from a Gitter staining sample after treatment, and the ratio of the dots for reticular fiber against entire dots in each field (percentage of reticulin fibrosis area) was measured as an index of the reticular fiber hyperplasia. It was confirmed that the reticular fiber hyperplasia in bone marrow was significantly improved in the mice which had been administered VA-Lip-siRNA HSP47-D (VA-siRNA D) compared to the untreated mice (No treatment), Lip-siRNA HSP47-administered mice (siRNA D) and VA-Lip-siRNA Random-administered mice (VA-siRNA ran) (see, Fig. 12).

Moreover, from the Azan staining samples after treatment as shown in Fig. 13, it was shown that the collagen hyperplasia in bone marrow was significantly improved in the two mice which had been administered VA-Lip-siRNA HSP47 (VA-Lip-siRNA HSP47 (1) and (2)) compared to the untreated mice (No treatment), Lip-siRNA HSP47-administered mice (Lip-siRNA HSP47) and VA-Lip-siRNA Random-treated mice (VA-Lip-siRNA ran).
Furthermore, from the HE staining samples after treatment as shown in Fig. 14, it was shown that the trabecular thickening was significantly improved in the two mice which had been administered VA-Lip-siRNA HSP47 (VA-Lip-siRNA HSP47 (1) and (2)) compared to the untreated mice (No treatment), Lip-siRNA HSP47-administerd mice (Lip-siRNA HSP47) and VA-Lip-siRNA Random-administered mice (VA-Lip-siRNA ran).
From these results, it has been suggested that the treatment for myelofibrosis using an siRNA targeted to HSP47 would be useful. Also, in view of the fact that siRNAs basically act in cytoplasm, the results above suggest that a retinoid functioned as a targeting agent to an extracellular matrix-producing cell in bone marrow and efficiently delivered a drug to this cell, and thereby significantly improving the pathology of myelofibrosis.

## Claims

1. A composition for use in the treatment of myelofibrosis, comprising a drug that controls the activity or growth of an extracellular matrix-producing cell in bone marrow, and a carrier comprising a retinoid as a targeting agent, wherein the drug that controls the activity or growth of the extracellular matrix-producing cell in bone marrow is selected from the group consisting of a drug that suppresses the expression of the extracellular matrix component and is selected from an siRNA, a ribozyme and antisense nucleic acid or a vector expressing these or an siRNA, a ribozyme, an antisense nucleic acid which target molecules involved in the production or secretion of said extracellular matrix component, and a vector that expresses said siRNA, ribozyme, and antisense nucleic acid.

2. The composition for use according to Claim 1, wherein the molecule involved in the production or secretion of the extracellular matrix component is HSP47.

3. The composition for use according to Claim 1 or 2, wherein the retinoid comprises retinol.

4. The composition for use according to any one of Claims 1 to 3, wherein the retinoid content is 0.2 - 20 wt % of the entire carrier.

5. The composition for use according to any one of Claims 1 to 4, wherein the carrier has a form, which is selected from the group consisting of a macromolecular micelle, a liposome, an emulsion, microspheres and nanospheres.

6. The composition for use according to any one of Claims 1 to 5, wherein the carrier has a form of a liposome, and the molar ratio of the retinoid to the lipid contained in the liposome is 8:1 - 1:4.

7. The composition for use according to any one of Claims 1 to 6, which is provided in a form that can be prepared immediately before use.

8. A kit for preparing a composition for use according to any one of Claims 1 to 7, comprising one or more containers that comprises either singly or in combination the drug that controls the activity or growth of an extracellular matrix-producing cell in bone marrow, the retinoid, and if necessary, a carrier-constituent substance other than the retinoid,
further comprising instructions for administrating the composition as specified in Claim 1,
wherein the drug that controls the activity or growth of the extracellular matrix-producing cell in bone marrow is selected from the group consisting of a drug that suppresses the expression of the extracellular matrix component and is selected from an siRNA, a ribozyme and antisense nucleic acid or a vector expressing these or an siRNA, a ribozyme, an antisense nucleic acid which target molecules involved in the production or secretion of said extracellular matrix component, and a vector that expresses said siRNA, ribozyme, and antisense nucleic acid.

## Patentansprüche

1. Zusammensetzung zur Verwendung bei der Behandlung von Myelofibrose, umfassend einen Wirkstoff, der die Wachstumsaktivität einer Zelle im Knochenmark, die extrazelluläre Matrix produziert, kontrolliert, und einen Träger, der ein Retinoid als Targeting-Wirkstoff umfasst, wobei der Wirkstoff, der die Wachstumsaktivität der Zelle im Knochenmark, die extrazelluläre Matrix produzieret, kontrolliert, ausgewählt wird aus der Gruppe, bestehend aus einem Wirkstoff, der die Expression der Komponente der extrazellulären Matrix supprimiert und ausgewählt wird aus einer siRNA, einem Ribozym und einer Antisens-Nukleinsäure oder einem Vektor, der diese exprimiert oder einer siRNA, einem Ribozym, einer Antisens-Nukleinsäure, die auf Moleküle, die in der Produktion oder Sekretion der besagten Komponente der extrazellulären Matrix involviert sind, abzielen und einem Vektor, der die besagten siRNA, Ribozym und Antisens-Nukleinsäure exprimiert.

2. Zusammensetzung zur Verwendung gemäß Anspruch 1, wobei das Molekül, das in der Produktion oder Sekretion der Komponente der extrazellulären Matrix involviert ist, HSP47 ist.

3. Zusammensetzung zur Verwendung gemäß Anspruch 1 oder 2, wobei das Retinoid Retinol umfasst.

4. Zusammensetzung zur Verwendung gemäß einem der Ansprüche 1 bis 3, wobei der Gehalt der Retinoid-Komponente im gesamten Träger 0,2 bis 20 Gew.% beträgt.

5. Zusammensetzung zur Verwendung gemäß einem der Ansprüche 1 bis 4, wobei der Träger in einer Form, ausgewählt aus der Gruppe, bestehend aus einer makromolekularen Micelle, einem Liposom, einer Emulsion, Mikrosphären und Nanosphären, vorliegt.

6. Zusammensetzung zur Verwendung gemäß einem der Ansprüche 1 bis 5, wobei der Träger in Form eines Liposoms vorliegt und das Molverhältnis des Retinoids zu dem in dem Liposom enthaltenen Lipid 8:1 bis 1:4 beträgt.

7. Zusammensetzung zur Verwendung gemäß einem der Ansprüche 1 bis 6, die in einer Form bereitgestellt wird, die unmittelbar vor Gebrauch zubereitet werden kann.

8. Kit zur Zubereitung einer Zusammensetzung zur Verwendung gemäß einem der Ansprüche 1 bis 7, umfassend einen oder mehrere Behälter, der/die entweder einzeln oder in Kombination den Wirkstoff, der die Wachstumsaktivität einer Zelle im Knochenmark, die extrazelluläre Matrix produziert, kontrolliert, das Retinoid und, falls erforderlich, eine vom Retinoid unterschiedliche Trägerbestandteil-Substanz, umfasst,
ferner umfassend Anweisungen zur Verabreichung der gemäß Anspruch 1 beschriebenen Zusammensetzung,
wobei der Wirkstoff, der die Wachstumsaktivität der Zelle im Knochenmark, die extrazelluläre Matrix produziert, kontrolliert, ausgewählt wird aus der Gruppe, bestehend aus einem Wirkstoff, der die Expression der Komponente der extrazellulären Matrix supprimiert und ausgewählt wird aus einer siRNA, einem Ribozym und einer Antisens-Nukleinsäure oder einem Vektor, der diese exprimiert oder einer siRNA, einem Ribozym, einer Antisens-Nukleinsäure, die auf Moleküle, die in der Produktion oder Sekretion der besagten Komponente der extrazellulären Matrix involviert sind, abzielen und einem Vektor, der die besagten siRNA, Ribozym und Antisens-Nukleinsäure exprimiert.

## Revendications

1. Composition destinée à être utilisée dans le traitement de la myélofibrose, comprenant un médicament qui supprime l'activité ou la croissance d'une cellule produisant une matrice extracellulaire dans la moelle osseuse, et un support comprenant un rétinoïde en tant qu'agent de ciblage, dans laquelle le médicament qui régule l'activité ou la croissance d'une cellule produisant une matrice extracellulaire dans la moelle osseuse est choisi dans le groupe constitué d'un médicament qui supprime l'expression du composant de matrice extracellulaire et est choisi parmi un ARNsi, un ribozyme et un acide nucléique antisens ou un vecteur les exprimant ou un ARNsi, un ribozyme, un acide nucléique antisens qui ciblent les molécules impliquées dans la production ou la sécrétion dudit composant de matrice extracellulaire, et un vecteur qui exprime lesdits ARNsi, ribozyme et acide nucléique antisens.

2. Composition pour utilisation selon la revendication 1, dans laquelle la molécule impliquée dans la production ou la sécrétion du composant de matrice extracellulaire est HSP47.

3. Composition pour utilisation selon la revendication 1 ou 2, dans laquelle le rétinoïde comprend le rétinol.

4. Composition pour utilisation selon l'une quelconque des revendications 1 à 3, dans laquelle la teneur en rétinoïde est de 0,2 % à 20 % en poids du support entier.

5. Composition pour utilisation selon l'une quelconque des revendications 1 à 4, dans laquelle le support a une forme qui est choisie dans le groupe constitué d' une micelle macromoléculaire, d'un liposome, d'une émulsion, de microsphères et de nanosphères.

6. Composition pour utilisation selon l'une quelconque des revendications 1 à 5, dans laquelle le support a la forme d'un liposome et le rapport molaire du rétinoïde au lipide contenu dans le liposome est de 8/1 à 1/4.

7. Composition pour utilisation selon l'une quelconque des revendications 1 à 6, qui est fournie sous une forme qui peut être préparée immédiatement avant utilisation.

8. Kit de préparation d'une composition pour utilisation selon l'une quelconque des revendications 1 à 7, comprenant un ou plusieurs récipients qui comprennent soit individuellement, soit en combinaison, le médicament qui régule l'activité ou la croissance d'une cellule produisant une matrice extracellulaire dans la moelle osseuse, le rétinoïde et, si nécessaire, une substance constituante du support autre que le rétinoïde,
comprenant en outre des instructions pour l'administration de la composition telle que spécifiée dans la revendication 1,
dans lequel le médicament qui régule l'activité ou la croissance d'une cellule produisant une matrice extracellulaire dans la moelle osseuse est choisi dans le groupe constitué d' un médicament qui supprime l'expression du composant de matrice extracellulaire et est choisi parmi un ARNsi, un ribozyme et un acide nucléique antisens ou un vecteur les exprimant ou un ARNsi, un ribozyme, un acide nucléique antisens qui ciblent les molécules impliquées dans la production ou la sécrétion dudit composant de matrice extracellulaire, et un vecteur qui exprime lesdits ARNsi, ribozyme et acide nucléique antisens.
